# EUROPÄISCHE PATENTSCHRIFT

(11) **EP 1 165 638 B1**
(45) Veröffentlichungstag und Bekanntmachung des Hinweises auf die Patenterteilung: **07.04.2004**
(21) Anmeldenummer: 00906246.4
(22) Anmeldetag: 29.01.2000
(51) Int. Cl.: C08F 220/06, C08F 8/00, A61L 15/00, A61F 13/15

(54) **VERNETZTE, HYDROPHILE, HOCHQUELLFÄHIGE HYDROGELE, VERFAHREN ZU IHRER HERSTELLUNG UND IHRE VERWENDUNG**
CROSS-LINKED, HYDROPHILIC HIGHLY SWELLABLE HYDROGELS, METHOD FOR THEIR PRODUCTION AND THEIR USE
HYDROGELS RETICULES, HYDROPHILES, A FORTE CAPACITE DE GONFLEMENT, ET PROCEDE DE PREPARATION ET D'UTILISATION DESDITS HYDROGELS

(30) Priorität: 05.02.1999 US 245298
(43) Veröffentlichungstag der Anmeldung: 02.01.2002
(73) Patentinhaber: BASF AKTIENGESELLSCHAFT, 67056 Ludwigshafen (DE)
(72) Erfinder: FUNK, Rüdiger, D-65527 Niedernhausen (DE); HERFERT, Norbert, D-63674 Altenstadt (DE); WANIOR, Mariola, D-63526 Erlensee (DE); BROWN, Patricia, D., Portsmouth, VA 23701 (US); ENGELHARDT, Friedrich, Chesapeake, VA 23320 (US); WOODRUM, Guy, T., Chesapeake, VA 23703 (US)
(86) Internationale Anmeldenummer: PCT/EP2000/000703
(87) Internationale Veröffentlichungsnummer: WO 2000/046260

(56) Entgegenhaltungen:
- EP-A- 0 615 736
- EP-A- 0 690 077
- WO-A-94/09043
- WO-A-98/47454
- US-A- 5 149 335
- US-A- 5 562 646
- US-A- 5 747 570
- US-A- 5 837 789

## Beschreibung

Die vorliegende Erfindung betrifft vernetzte, hydrophile, hochquellfähige Hydrogele, die einen Pressure Absorbency Index < 100 und eine Vertikalabsorption unter Druck von 1922,8 Pa von mindestens 12 g/g aufweisen, ein Verfahren zu ihrer Herstellung und ihre Verwendung.

Hydrophile Hydrogele, die durch Polymerisation ungesättigter Säuren, wie beispielsweise Acrylsäure, Methacrylsäure oder Acrylamidopropansulfonsäure in Gegenwart geringer Mengen mehrfach olefinisch ungesättigter Verbindungen erhalten werden können, sind bereits als superabsorbierende Polymere bekannt. Sie werden beispielsweise beschrieben in US-A-4,057,521, US-A-4,062,817, US-A-4,525,527, US-A-4,286,082, US-A-4,340,706 und US-A-4,295,987.

Weiterhin sind hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind vgl. z. B. US-A-5,011,892, US-A-4,076,663 und US-A-4,931,497).

Die genannten Hydrogele zeichnen sich durch ein hohes Aufnahmevermögen für Wasser und wäßrige Lösungen aus und finden daher als superabsorbierende Polymere bevorzugt Anwendung als Absorptionsmittel in Hygieneartikeln.

Der zunehmenden Tendenz, Hygieneartikel wie Baby- und Inkontinenz-Windel immer kleiner und dünner zu gestalten, kann bei Beibehaltung der Gesamt-Absorptionskapazität nur dadurch entsprochen werden, daß man den großvolumigen Fluffanteil reduziert und den Anteil an hochquellfähigem Hydrogel erhöht. Hierdurch müssen die superabsorbierende Polymere zusätzliche Aufgaben bezüglich Flüssigkeitsaufnahme, -transport und -verteilung übernehmen, die vorher der Fluff erfüllte, um eine Leckage des Hygieneartikels aufgrund von Gel-blocking-Phänomenen zu verhindern.

Aus der US-A-5,147,343 sind absorbierende Zusammensetzungen bekannt, die aus einer porösen Faser-Matrix und superabsorbierendem Polymer bestehen, das in den Poren der Faser-Matrix dispergiert ist. Das superabsorbierende Polymer ist gekennzeichnet, daß es unter einer Druckbelastung von 21000 dynes/cm² mindestens 27 ml einer 0,9 Gew.-%igen Natriumchlorid-Lösung absorbiert. Der Anteil an superabsorbierendem Polymer in der absorbierenden Zusammensetzung beträgt bevorzugt 10 - 60 Gew.-%.

Die US-A-5,149,335 beschreibt absorbierende Strukturen mit einem Gehalt an superabsorbierendem Polymer von 60 - 100 Gew.-%. Das superabsorbierende Polymer ist gekennzeichnet durch eine Free-Swell Rate von kleiner 60 s und einem 5-Minuten AUL-Wert (Absorption unter Belastung) von mindestens 15 g/g.

Aus der EP-A-O 532 002 sind absorbierende Zusammensetzungen bekannt, die aus einer porösen Faser-Matrix und mindestens 30 Gew.-% superabsorbierendem Polymer bestehen, wobei das superabsorbierende Polymer eine Deformation Under Load von 0,60 mm oder weniger und einen Wicking Index von 10 cm oder mehr aufweist.

Die EP-A-O 615 736 betrifft absorbierende Zusammensetzungen mit einem Gehalt von 30 - 100 Gew.-% an superabsorbierendem Polymer mit einem Pressure Absorbency Index von mindestens 100 und einem Gehalt an extrahierbaren Anteilen (16 h Extraktion in 0,9 gew.-%iger wäßriger Kochsalzlösung) von weniger als 13 Gew.-%.

Die EP-A-O 761 191 beschreibt absorbierende Zusammensetzungen bestehend aus einer Faser-Matrix und mindestens 30 Gew.-% superabsorbierendem Polymer, welches einen Wicking Parameter von 700 oder größer hat.

Aus der US-A-5,562,646 ist eine absorbierende Zusammensetzung bekannt, die mindestens eine Region mit einem superabsorbierendem Polymeren in einer Konzentration von 60 - 100 Gew.-% enthält, wobei das superabsorbierende Polymer eine Porosität von mindestens 0,15 und eine Performance Under Pressure unter einer Druckbelastung von 0,7 psi (4826,5 Pa) von mindestens 23 g/g aufweist.

US-A-5,599,335 und US-A-5,669,894 beschreiben absorbierende Zusammensetzungen, die mindestens eine Region mit einem superabsorbierendem Polymeren in einer Konzentration von 60 - 100 Gew.-% enthalten, wobei das superabsorbierende Polymer einen Saline Flow Conductivity-Wert von mindestens 30 x 10⁻⁷ cm³sec/g und eine Performance Under Pressure unter einer Druckbelastung von 0.7 psi von mindestens 23 g/g aufweist.

Wenngleich durch den Einsatz der in dem Stand der Technik beschriebenen superabsorbierenden Polymeren die Qualität von mit Hydrogel hochbeladenen Hygieneartikeln im Vergleich zum Einsatz von schwach vernetzten, nicht-oberflächennachvernetzten Superabsorber-Produkten der sogenannten 1. Generation verbessert worden ist, zeigen die bisher bekannten superabsorbierenden Polymeren in diesen Hygieneartikeln bezüglich Flüssigkeitsaufnahme, -transport und -verteilung jedoch noch durchweg Nachteile, so daß einer Erhöhung des Anteils an hochquellfähigem Hydrogel in der Windel durch den dadurch eintretenden Qualitätsverlust Grenzen gesetzt sind.

Der vorliegenden Erfindung liegt daher die Aufgabe zugrunde, ein hochquellfähiges Hydrogel bereitzustellen, das die zuvor geschilderten Nachteile nicht aufweist und auch bei Verwendung in Windelkonstruktionen mit sehr hohem Anteil an superabsorbierendem Polymer ein ausgezeichnetes Aufnahme- und Rückhaltevermögen von Körperflüssigkeiten zeigt.

Erfindungsgemäß erfolgt die Lösung dieser Aufgabe durch ein vernetztes, hydrophiles, hochquellfähiges Hydrogels auf Basis polymerisierter Monomerer oder auf Basis von Pfropfpolymeren, wenn das Hydrogel einen Pressure Absorbency Index < 100 und eine Vertikalabsorption unter Druck von 1922,8 Pa von mindestens 12 g/g aufweist. Besonders bevorzugt sind hierbei Hydrogele, die eine Performance Under Pressure unter einer Druckbelastung von 0,7 psi (4826,5 Pa) von kleiner 23 g/g und/oder eine Absorbency Under Load (AUL) unter einer Druckbelastung von 21000 dynes/cm² (2100 Pa) von kleiner 27 g/g aufweisen. Die extrem hohe Vernetzung der Hydrogele erreicht man mit einem Verfahren zur Herstellung der vernetzten, hydrophilen, hochquellfähigen Hydrogele dadurch, daß man Säuregruppen enthaltende hydrophile Monomere, deren Alkalimetall- oder Ammoniumsalze mit
(a) einem Copolymerisationsvernetzer, der mindestens zwei ethylenisch ungesättigte Doppelbindungen im Molekül enthält, und
(b) einem Reaktivvernetzer A, der eine ethylenisch ungesättigte Doppelbindung und mindestens eine funktionelle Gruppe aufweist, die mit den Säuregruppen der hydrophilen Polymerisate kovalente Bindungen bildet, einem Reaktivvernetzer B, der mindestens zwei funktionelle Gruppen aufweist, die mit den Säuregruppen der hydrophilen Polymerisate kovalente Bindungen bilden, und/oder mit Ionen mehrwertiger Metalle
gegebenenfalls in Gegenwart mindestens einer Pfropfgrundlage, radikalisch zu einem vernetzten, hydrophilen Grundpolymer polymerisiert, das Grundpolymer zerkleinert und die Oberfläche der Partikeln des Grundpolymers nachvernetzt. Durch eine Oberflächennachvernetzung der zunächst erhaltenen Grundpolymere wird die Vernetzungsdichte der Oberfläche nochmals erhöht.

Die erfindungsgemäßen hochvernetzten, hydrophilen, hochquellfähigen Hydrogele sowie das Verfahren zu ihrer Herstellung werden im folgenden näher erläutert.

Zur Herstellung der erfindungsgemäßen, wasserquellbaren hydrophilen Polymeren geeignete hydrophile Monomere sind beispielsweise polymerisationsfähige Säuren, wie Acrylsäure, Methacrylsäure, Vinylsulfonsäure, Vinylphoshonsäure, Styrolsulfonsäure, Maleinsäure einschließlich deren Anhydrid, Furmarsäure, Itaconsäure, 2-Acrylamido-2-methylpropan-sulfonsäure, 2-Acrylamido-2-methylpropanphosphonsäure sowie deren Amide, Hydroxy-alkylester und aminogruppen- oder ammoniumgruppenhaltige Ester und Amide. Desweiteren wasserlösliche N-Vinylamide oder auch Diallyldimethylammoniumchlorid.

Bevorzugte hydrophile Monomere sind Verbindungen der allgemeinen Formel (I) 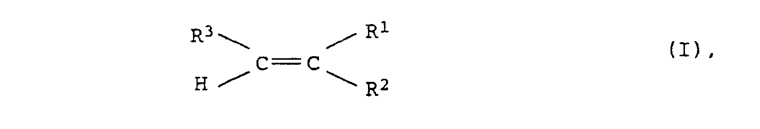 worin
- R¹: Wasserstoff, Methyl oder Ethyl,
- R²: die Gruppe -COOR⁴, die Sulfonylgruppe, die Phosphonylgruppe, die mit (C₁ - C₄)-Alkanol veresterte Phosphonylgruppe oder eine Gruppe der Formel 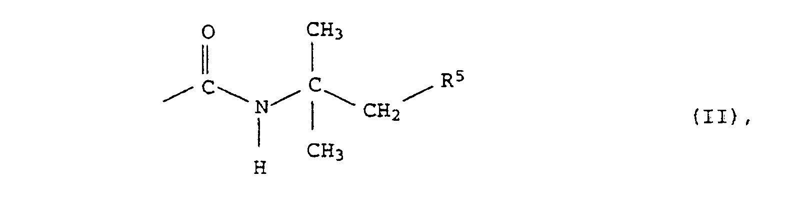
- R³: Wasserstoff, Methyl, Ethyl oder die Carboxylgruppe,
- R⁴: Wasserstoff, Alkalimetall- oder Ammoniumion und
- R⁵: Sulfonyl-, Phosphonyl- oder Carboxylgruppe bedeuten.

Beispiele für (C₁ - C₄)-Alkanole sind Methanol, Ethanol, n-Propanol, Isopropanol oder n-Butanol.

Besonders bevorzugte hydrophile Monomere sind Acrylsäure und Methacrylsäure.

Geeignete Pfropfgrundlagen können natürlichen oder synthetischen Ursprungs sein. Beispiele sind Stärke, Cellulose oder Cellulosederivate sowie andere Polysaccharide und Oligosaccharide, Polyvinylalkohol, Polyalkylenoxide, insbesondere Polyethylenoxide und Polypropylenoxide oder Blockcopolymerisate aus Ethylenoxid und Propylenoxid sowie hydrophile Polyester. Geeignete Polyalkylenoxide haben beispielsweise die Formel 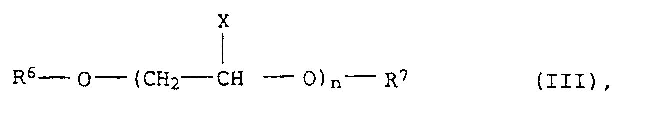 worin
- R⁶: und R⁷ unabhängig voneinander Wasserstoff, Alkyl, Alkenyl oder Aryl,
- X: Wasserstoff oder Methyl und
- n: eine ganze Zahl von 1 bis 10000 bedeuten.

R⁶und R⁷ stehen bevorzugt für Wasserstoff, (C₁ - C₄)-Alkyl, (C₂ - C₆)-Alkenyl oder Phenyl.

Pro 100 Gewichtsteile der bei der Polymerisation eingesetzten Monomeren verwendet man z.B. 0 bis 30, vorzugsweise 0 bis 10 Gewichtsteile mindestens einer Pfropfgrundlage.

Die Herstellung der erfindungsgemäßen hochquellfähigen Hydrogele erfolgt durch den gemeinsamen Einsatz von Copolymerisations- und Reaktivvernetzern und/oder Ionen mehrwertiger Metalle.

Copolymerisationsvernetzer sind Verbindungen mit mindestens zwei Doppelbindungen im Molekül, die mit den zur Herstellung der erfindungsgemäßen, wasserquellbaren hydrophilen Polymeren geeigneten hydrophilen Monomeren copolymerisierbar sind. Geeignete Copolymerisationsvernetzer sind insbesondere Methylenbisacryl- bzw. -methacrylamid, Ester ungesättigter Mono- oder Polycarbonsäuren von Polyolen, wie Diacrylat oder Triacrylate, z. B. Butandiol- oder Ethylenglykoldiacrylat bzw. -methacrylat sowie Trimethylolpropantriacrylat, Allylverbindungen wie Allyl(meth)acrylat, Triallylcyanurat, Maleinsäurediallylester, Polyallylester, Tetraallyloxyethan, Triallylamin, Tetraallylethylendiamin, Pentaerythritoltriallylether oder Allylester der Phosphorsäure sowie Vinylverbindungen wie Vinylacrylat, Divinyladipinat, Divinylbenzol und Vinylphosphonsäurederivate, wie sie beispielsweise in der EP-A 343 427 beschrieben sind. Bevorzugt ist der Einsatz von Allyl- und (Meth)acrylat-Vernetzern. Diese Vernetzer werden beispielsweise in Mengen von 0,1 bis 5,0, vorzugsweise 0,5 bis 3,0 Gew.-%, bezogen auf die hydrophilen Monomeren, bei der Polymerisation eingesetzt.

Reaktivvernetzer B sind Verbindungen, die mindestens zwei funktionelle Gruppen enthalten, die mit den funktionellen Gruppen der einpolymerisierten hydrophilen Monomeren, beispielsweise den Carboxylgruppen der Acrylsäure, unter Ausbildung von ionischen und/oder kovalenten Bindungen reagieren. Beispiele für solche Verbindungen sind mehrwertige Alkohole, mehrwertige Amine, Polyamidoamine und deren Umsetzungsprodukte mit Epichlorhydrin, Di- und Polyepoxide, Bis- und Polyaziridine, Bis- und Polyoxazoline, Di- und Polyisocyanate, Ethylen- und Propylencarbonat, 2-Oxazolidon und dessen Derivate, Polyethylenimine, Poly(diallyldimethylammonium)chlorid, Polyvinylamine, sowie alle Salze mehrwertiger Metallionen. Bevorzugt ist der Einsatz von Polyamidoaminen und deren Umsetzungsprodukte mit Epichlorhydrin oder mit Bischlorhydrinethern von Polyethylenglykolen, Polypropylenglykole oder Blockcopolymerisaten aus Ethylenoxid und Propylenoxid mit Molmassen bis zu jeweils 6000 sowie von Aluminiumsalzen wie z. B. Natriumaluminat. Die Reaktivvernetzer B werden bei der Polymerisation beispielsweise in Mengen von 0,05 bis 7,5 Gew.-%, bezogen auf die hydrophilen Monomeren bzw. nach der Polymerisation und einem Zerkleinern der erhaltenen hydrophilen Gele in Mengen von beispielsweise 0,02 bis 3,0, vorzugsweise 0,04 bis 2,0 Gew.-%, bezogen auf die Polymerisate, eingesetzt.

Desweiteren ist es auch möglich, Verbindungen einzusetzen, die sowohl den Charakter eines Copolymerisationsvernetzers als auch eines Reaktivvernetzers aufweisen. Diese Verbindungen werden nachstehend Reaktivvernetzer A genannt. Diese Verbindungen haben mindestens eine Doppelbindung im Molekül, die mit den zur Herstellung der erfindungsgemäßen, wasserquellbaren hydrophilen Polymeren geeigneten hydrophilen Monomeren eine Copolymerisation eingehen kann, und mindestens eine funktionellen Gruppe, die mit den funktionellen Gruppen der zur Herstellung der erfindungsgemäßen, wasserquellbaren hydrophilen Polymeren geeigneten hydrophilen Monomeren, beispielsweise den Carboxylgruppen der Acrylsäure, unter Ausbildung von ionischen und/oder kovalenten Bindungen reagieren kann. Beispiele für solche Verbindungen sind Glycidylmethacrylat oder 2-Hydroxyethyl(meth)acrylat. Diese Gruppe von Vernetzern wird bei der Herstellung der Polymerisate eingesetzt. Bezogen auf die bei der Polymerisation eingesetzten hydrophilen Monomeren verwendet man beispielsweise 0,1 bis 5,0 Gew.-% an Vernetzern A, die eine Doppelbindung und mindestens eine funktionelle Gruppe enthalten, die mit den Säuregruppen der hydrophilen Polymerisate eine kovalente Bindung bilden kann.

In einer bevorzugten Ausführungsform des erfindungsgemäßen Verfahrens zur Herstellung der vernetzten, hydrophilen, hochquellfähigen Hydrogele polymerisiert man Säuregruppen enthaltende hydrophile Monomere, deren Alkalimetall- oder Ammoniumsalze mit
(a) einem Copolymerisationsvernetzer, der mindestens zwei ethylenisch ungesättigte Doppelbindungen im Molekül enthält, und gegebenenfalls
(b) einem Reaktivvernetzer A, der eine ethylenisch ungesättigte Doppelbindung und mindestens eine funktionelle Gruppe aufweist, die mit den Säuregruppen der hydrophilen Polymerisate kovalente Bindungen bildet,
gegebenenfalls in Gegenwart einer Pfropfgrundlage, radikalisch zu einem vernetzten, hydrophilen Grundpolymer, zerkleinert das Grundpolymer, mischt es während des Zerkleinerns oder danach mit einem Reaktivvernetzer B und/oder mit Ionen mehrwertiger Metalle und führt eine Nachvernetzung der Oberfläche der Partikeln des Grundpolymers durch. Für die Nachvernetzung der Oberfläche der Partikeln des Grundpolymers setzt man mit Vorteil Natriumaluminat ein.

Zur Auslösung der Polymerisation können energiereiche elektromagnetische Strahlen oder die üblichen chemischen Polymerisationsinitiatoren herangezogen werden, z. B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie Ammoniumpersulfat, Kaliumpersulfat oder Wasserstoffperoxid, gegebenenfalls in Kombination mit Reduktionsmitteln wie Natriumhydrogensulfit, und Eisen(II)-sulfat oder Redoxsysteme, die als reduzierende Komponente eine aliphatische und aromatische Sulfinsäure, wie Benzolsulfinsäure und Toluolsulfinsäure oder Derivate dieser Säure enthalten, z.B. Addukte aus Sulfinsäure, Aldehyden und Aminoverbindungen.

Bevorzugt ist die Polymerisation in wäßriger Lösung nach der sogenannten Gel-Polymerisation unter Ausnutzung des Trommsdorff-Norrish-Effektes, vgl. Makromol. Chem. 1, 169 (1947))). Die Polymerisation kann hierbei sowohl diskontinuierlich als auch kontinuierlich durchgeführt werden.

Während die Zugabe der Copolymerisationsvernetzer und der Vernetzer A, die eine Doppelbindung und mindestens eine reaktive Gruppe enthalten, stets vor der Initiierung oder während der Polymerisation zur Monomerlösung erfolgen muß, kann die Zugabe der Reaktivvernetzer B und der Ionen mehrwertiger Metalle entweder zur Monomerlösung oder nach Abschluß der Polymerisation erfolgen. Im letzteren Falle wird das Gel vorteilhafterweise zunächst in geeigneten Vorrichtungen zerkleinert und anschließend beispielsweise in einem Kneter oder einem Wolf mit den Reaktivvernetzern B und/oder Ionen mehrwertiger Metalle umgesetzt. Als Copolymerisationsvernetzer setzt man vorzugsweise Verbindungen mit mindestens zwei Allyl-, Methacrylat- und/oder Acrylatgruppen ein. Bevorzugt verwendete Reaktivvernetzer B sind beispielsweise Polyamidoamine, deren Umsetzungsprodukte mit Epichlorhydrin oder Bischlorhydrinethern von Alkylenglykolen oder Polyalkylenglykolen. Von Interesse ist außerdem der Einsatz von Natriumaluminat als Vernetzerkomponente (b). Bevorzugt ist eine Verfahrensvariante, bei der die Copolymerisation der Säuregruppen enthaltenden hydrophilen Monomeren in Gegenwart von
(a) Allylmethacrylat, Tetraallyloxethan, Methylenbisacrylamid, Petaerythrittriallylether oder deren Mischungen und
(b) Hydroxyethylacrylat, Hydroxyethylmethacrylat, Hydroxypropylacrylat, Hydroxypropylmethacrylat, Hydroxybutylacrylaten oder Mischungen der genannten Verbindungen
als Vernetzer durchgeführt wird.

Die Trocknung der Hydrogelteilchen erfolgt nach Verfahren, die dem Fachmann bekannt sind, beispielsweise nach dem Drehtrommel-Verfahren mit Hilfe von Walzentrocknern oder nach dem Förderband-Verfahren, bei der mit Löcher versehene Horden eines Kreisförderes in einem Tunnel mit Trocknungsgut beladen und das Trocknungsgut während der Förderung durch Durchblasen von Heißluft durch die Hordenlöcher getrocknet wird.

Die Korngrößenverteilung des getrockneten und gegebenfalls vorzerkleinerten Hydrogels wird durch Mahlung eingestellt, wobei die Partikelgröße der Hydrogel-Teilchen in der Regel zwischen 50 und 2000 µm, bevorzugt zwischen 100 und 1000 µm, liegt.

Zur Herstellung der erfindungsgemäßen, hochvernetzten, hydrophilen hochquellfähigen Hydrogele wird das zunächst erhaltene vernetzte Grundpolymer-Hydrogel einer anschließenden Oberflächennachvernetzung unterworfen. Hierzu werden Verbindungen, die mit den funktionellen Gruppen des Hydrogels unter Vernetzung reagieren können (Reaktivvernetzer B und Ionen mehrwertiger Metalle), vorzugsweise in Form einer wasserhaltigen Lösung auf die Oberfläche der Hydrogel-Partikeln aufgebracht. Geeignete Nachvernetzungsmittel wurden bereits bei den Reaktivvernetzern genannt. Es handelt sich beispielsweise um Di- oder Polyglycidylverbindungen wie Phosphonsäurediglycidylether oder Ethylenglykoldiglycidylether, Alkoxysilylverbindungen, Polyaziridine, Polyamine oder Polyamidoamine sowie deren Umsetzungsprodukte mit Epichlorhydrin, Polyole wie Ethylenglykol, 1,2-Propandiol, 1,4-Butandiol, Glycerin, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat, Oxazolidon und dessen Derivate, Bisoxazolin, Polyoxazoline, Di- und Polyisocyanate. Bei Bedarf können saure Katalysatoren wie beispielsweise p-Toluolsulfonsäure, Phosphorsäure, Borsäure oder Ammoniumdihydrogenphosphat zugesetzt werden. Bei der Nachvernetzung setzt man z.B. pro 100 Gew.-Teile Grundpolymer-Hydrogel 0,001 bis 5,0, vorzugsweise 0,01 bis 1,0 Gew.-% mindestens eines der genannten Nachvernetzungsmittel ein. Bevorzugt verwendete Nachvernetzungsmittel sind Diglycidylether, Umsetzungsprodukte von Polyamidoaminen mit Epichlorhydrin, Bischlorhyrinether von Alkylenglykolen oder Polyalkylenglykolen, Polyethyleniminen, Vinylamineinheiten enthaltenden Polymeren oder deren Mischung.

Geeignete Mischaggregate zum Aufsprühen der Vernetzer-Lösung auf die Hydrogel-Partikel sind beispielsweise Patterson-Kelly-Mischer, DRAIS-Turbulenzmischer, Lödige-Mischer, Schneckenmischer, Tellermischer, Wirbelschichtmischer, Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt 80 - 190 °C, und besonders bevorzugt zwischen 100 und 160 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können.

In einer besonderen Ausführung der Erfindung wird zusätzlich die Hydrophilie der Hydrogel-Partikeloberfläche durch Ausbildung von Metallkomplexen modifiziert. Die Bildung der Metallkomplexe auf der äußeren Schale der Hydrogel-Partikel erfolgt durch Aufsprühen von Lösungen zwei- oder mehrwertiger Metallsalze, wobei die Metall-Kationen mit den funktionellen Gruppen des Hydrogels unter Ausbildung von Komplexen reagieren können. Beispiele für zwei- oder mehrwertige Metall-Kationen sind Mg²⁺, Ca²⁺, Al³⁺, Sc³⁺, Ti⁴⁺, Mn²⁺, Fe^{2+/3+}, Co²⁺, Ni²⁺, Cu^{+/2+}, Zn²⁺, Y³⁺, Zr⁴⁺, Ag⁺, La³⁺, Ce⁴⁺, Hf⁴⁺, und Au^{+/3+}, bevorzugte Metall-Kationen sind Mg²⁺, Ca²⁺, Al³⁺, Ti⁴⁺, Zr⁴⁺ und La³⁺, und besonders bevorzugte Metall-Kationen sind Al³⁺, Ti⁴⁺ und Zr⁴⁺. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat. Als Lösungsmittel für die Metallsalze können eingesetzt werden Wasser, Alkohole, Dimethylformamid, Dimethylsulfoxid sowie Mischungen dieser Komponenten. Besonders bevorzugt sind Wasser und Wasser/Alkohol-Mischungen wie zum Beispiel Wasser/Methanol oder Wasser/1,2-Propandiol.

Das Aufsprühen der Metallsalz-Lösung auf die Hydrogel-Partikel kann sowohl vor als auch nach der Oberflächennachvernetzung der Hydrogel-Partikeln erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Optional kann noch eine weitere Modifizierung der Hydrogel-Partikel durch Zumischung feinteiliger anorganischer Feststoffe, wie zum Beispiel Silica, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Silica oder von Aluminiumoxid mit einer mittleren Größe der Primärteilchen von 4 bis 50 nm und einer spezifischen Oberfläche von 50 - 450 m²/g. Die Zumischung feinteiliger anorganischer Feststoffe erfolgt bevorzugt nach der Oberflächenmodifizierung durch Vernetzung /Komplexbildung, kann aber auch vor oder während dieser Oberflächenmodifizierungen durchgeführt werden.
Die Vernetzungsdichte der Hydrogele kann durch Messung der Absorptionskapazität ohne und unter Druckbelastung bestimmt werden. Eine hierfür besonders geeignete Methode stellt der sogenannte Pressure Absorbency Index dar, der in EP 0 615 736 beschrieben ist und die Summe der Werte für die Absorption unter 4 verschiedenen Belastungen (Absorption Under Load = AUL) darstellt, und zwar AUL 0,01 psi (69 Pa), AUL 0.29 psi (1999,5 Pa), AUL 0,57 psi (3930 Pa) und AUL 0,90 psi (6205,3 Pa). Weitere Methoden zur Messung der Absorptionskapazität ist die Performance Under Pressure (PUP) unter einer Druckbelastung von 0,7 psi (4826,5 Pa), wie sie in US-A-5,562,646 beschrieben wird, und die Absorbency Under Load (AUL) unter einer Druckbelastung von 21000 dynes/cm² (2100 Pa), wie sie in US-A-5,147,343 beschrieben ist.

Flüssigkeitstransport und -verteilung in einer gequollenen Hydrogelschicht können durch Messung der Vertikalabsorption unter Druck sowie durch Messung der Acquisition Time/Rewet unter Druck charakterisiert werden. Diese Testmethoden sind nachfolgend beschrieben.

Die erfindungsgemäßen Hydrogele weisen eine extrem hohe Vernetzung auf. Das Hydrogel besitzt einen Pressure Absorbency Index < 100 und gleichzeitig eine Vertikalabsorption unter Druck von mindestens 12 g/g. Besonders bevorzugt sind solche Hydrogele, die noch zusätzlich eine Performance Under Pressure unter einer Druckbelastung von 0,7 psi (4826,5 Pa) von kleiner 23 g/g und/oder eine Absorbency Under Load (AUL) unter einer Druckbelastung von 21000 dyn/cm² (2100 Pa) von kleiner 27 g/g aufweisen.

Die erfindungsgemäßen Hydrogele zeichnen sich durch hervorragende Eigenschaften bezüglich Flüssigkeitsaufnahme, -transport und -verteilung aus und sind deshalb in besonderer Weise als Absorptionsmittel für Wasser und wäßrige Flüssigkeiten, insbesondere von Körperflüssigkeiten, wie z. B. Urin oder Blut geeignet, beispielsweise in Hygieneartikeln wie z. B. Baby- und Erwachsenenwindeln, Damenbinden, Tampons und dergleichen. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

### Beschreibung der Testmethoden:

### Vertikalabsorption unter Druck:

Die zur Messung der Vertikalabsorption unter Druck benötigte Testapparatur besteht aus Meßzellen und einem Flüssigkeitsbehälter. Die Meßzellen stellen ein zylindrisches Plexiglas-Rohr mit einem Innendurchmesser von 2,6 cm und einer Länge von 15 cm dar. Das obere Ende des Rohres ist offen, das untere Ende des Rohres besitzt einen 36 µm-Siebboden. In Höhe von 3 cm (vom unteren Ende des Rohres) besitzt das Rohr einen Tragring. Der Flüssigkeitsbehälter ist ein Plexiglaskasten von 30,0 cm Länge, 20,5 cm Breite und 3,8 cm Höhe. Im Abstand von 1,5 cm von einer Querseite ist eine 2 cm hohe Überlaufwand eingebaut. Auf der gegenüberliegenden Seite befindet sich eine Verbindung zum Flüssigkeitsbehälter, so daß ein konstanter Flüssigkeitsspiegel gewährleistet ist. Der Plexiglaskasten besitzt einen abnehmbaren Deckel, der mit 6 kreisrunden Löchern mit einem Durchmesser von jeweils 3,2 cm versehen ist. Zur Durchführung der Messung werden 2 g Hydrogel in eine Meßzelle eingewogen, wobei die Hydrogel-Partikel gleichmäßig auf dem Siebboden verteilt werden. Anschließend werden die Hydrogel-Partikel mit einer wandgängigen Plexiglasscheibe überdeckt und ein wandgängiger Plexiglaszylinder mit Metallstiel eingeführt, wobei das Gesamtgewicht der Plexiglasscheibe und des Zylinders mit Stiel 100 g beträgt, so daß auf den Hydrogel-Partikeln ein Druck von 19,6 g/cm² lastet. Der Flüssigkeitsbehälter wird mit 0,9 Gew.-%iger Kochsalz-Lösung gefüllt. Dann wird die Meßzelle durch eine Bohrung des Deckels in die Flüssigkeit eingetaucht (Eintauchtiefe: 1,2 cm), wobei die Meßzelle durch den Tragring gehalten wird. Es können gleichzeitig bis zu 6 Meßzellen vermessen werden. Die Meßzellen werden 60 Minuten lang im Flüssigkeitsbehälter belassen, wobei die Hydrogel-Partikel unter Quellung Flüssigkeit entgegen der Schwerkraft unter zusätzlicher Gewichtsbelastung aufnehmen. Aufgrund der sehr hohen Flächenbelegung an Hydrogel-Partikeln ist zur Erzielung einer hohen Quellhöhe eine sehr gute Flüssigkeitsweiterleitung notwendig. Nach 60 Minuten wird die Meßzelle aus dem Flüssigkeitsbehälter genommen und die aufgenommene Flüssigkeitsmenge durch Auswiegen bestimmt. Die Vertikalabsorption unter Druck ergibt sich durch Division der aufgenommenen Flüssigkeitsmenge durch die Einwaage an Hydrogel.

### Acquisition Time/Rewet unter Druck:

Die Untersuchung wird an sogenannten Labor-Pads durchgeführt. Zur Herstellung dieser Labor-Pads werden 11,2 g Cellulose-Fluff und 23,7 g Hydrogel in einer Luft-Kammer homogen verwirbelt und durch Anlegen eines leichten Unterdruckes auf eine Form der Größe 12 x 26 cm abgelegt. Diese Zusammensetzung wird dann in Tissue-Papier eingeschlagen und bei einem Druck von 200 bar 2 mal 15 Sekunden lang verpreßt. Ein auf diese Weise hergestellter Labor-Pad wird auf einer waagerechten Unterlage befestigt. Die Mitte des Pads wird bestimmt und markiert. Die Aufgabe von synthetischer Harnersatzlösung erfolgt durch eine Kunststoffplatte mit einem Ring in der Mitte (Innendurchmesser des Ringes 6,0 cm, Höhe 4,0 cm). Die Platte wird belastet mit zusätzlichen Gewichten, so daß die Gesamtbelastung des Pads 13,6 g/cm2 beträgt. Die Kunststoffplatte wird auf dem Pad so plaziert, daß der Mittelpunkt des Pads gleichzeitig die Mitte des Aufgaberinges darstellt. Es werden dreimal 80 ml synthetische Harnersatzlösung aufgegeben. Die synthetische Harnersatzlösung wird hergestellt durch Auflösen von 1,26 g Magnesiumsulfat-Heptahydrat, 3,75 g Kaliumchlorid, 6,33 g Natriumchlorid, 15,00 g Harnstoff, 2,50 g Kaliumdihydrogenphosphat und 1,22 g Natriumhydrogenphosphat-Dihydrat in 1 kg E-Wasser. Die synthetische Harnersatzlösung wird in einem Meßzylinder abgemessen und durch den Ring in der Platte in einem Schuß auf den Pad aufgegeben. Gleichzeitig mit der Aufgabe wird die Zeit gemessen, die zum kompletten Eindringen der Lösung in den Pad notwendig ist. Die gemessene Zeit wird als Acquisition Time 1 notiert. Danach wird der Pad mit einer Platte für 20 Min belastet, wobei die Belastung weiterhin bei 13,6 g/cm2 gehalten wird. Nach dieser Zeit wird die Platte entfernt, auf den Mittelpunkt werden 10 g ±0,5 g vom Filterpapier (Schleicher & Schuell, 1450 CV) gelegt und mit einem Gewicht (Fläche 10 x 10 cm, Gewicht 3,5 kg) für 15 s belastet. Nach dieser Zeit wird das Gewicht entfernt, und das Filterpapier wird zurückgewogen. Der Gewichtsunterschied wird als Rewet 1 notiert. Danach wird die Kunststoffplatte mit Aufgabering erneut auf den Pad gelegt und die zweite Aufgabe der Flüssigkeit erfolgt. Die gemessene Zeit wird als Acquisition Time 2 notiert. Die Prozedur wird wiederholt wie beschrieben, aber für die Rewet Untersuchung werden 45g±0,5g Filterpapier verwendet. Der Rewet 2 wird notiert. Bei der Bestimmung von Acquisition Time 3 wird auf die gleiche Weise verfahren. Bei der Bestimmung von Rewet 3 werden 50g ±0,5 g Filterpapier gebraucht.

### Beispiele:

### Beispiel 1:

In einem durch geschäumtes Kunststoffmaterial gut isolierten Polyethylengefäß mit einem Fassungsvermögen von 10 1 werden 3600 g entsalztes Wasser und 1400 g Acrylsäure vorgelegt. Nun erfolgt Zugabe von 14 g Tetraallyloxyethan als Copolymerisationsvernetzer. Bei einer Temperatur von 4 °C werden die Initiatoren, bestehend aus 2,2 g 2,2'-Azobisamidino-propandihydrochlorid, gelöst in 20 g entsalztes Wasser, 4 g Kaliumperoxodisulfat, gelöst in 150 g entsalztes Wasser sowie 0,4 g Ascorbinsäure, gelöst in 20 g entsalztes Wasser, nacheinander zugegeben und verrührt. Die Reaktionslösung wird daraufhin ohne Rühren stehengelassen, wobei die Temperatur der Polymerisation bis auf ca. 92 °C ansteigt. Man erhält ein ein festes Gel, das anschließend mechanisch zerkleinert, durch Zugabe von 50 Gew.-%iger Natronlauge auf einen pH-Wert von 6,0 eingestellt und mit 150 g einer 15 Gew.-%igen wäßrigen Lösung eines Polyamidoamin-Epichlorhydrin-Adduktes (RETEN 204 LS der Fa. Hercules) als Reaktivvernetzer versetzt wird. Das Gel wird dann getrocknet, gemahlen und auf eine Korngrößenverteilung von 100 - 850 µm abgesiebt. 1 kg dieses getrockneten Hydrogels wird in einem Pflugscharmischer mit einer Lösung bestehend aus 40 g entsalztes Wasser, 40 g Methanol und 1,5 g Ethylenglykoldiglycidylether besprüht und anschließend für 60 Minuten bei 140 °C getempert. Das vorliegend beschriebene Produkt zeigt folgende Eigenschaften:
- Pressure Absorbency Index =: 90,2
- Vertikal-Absorption unter Druck =: 16,2 g/g
- PUP 0.7 psi (4826,5 Pa) =: 22,2 g/g
- AUL 21 dyn/cm² (2100 Pa) =: 22,3 g/g

### Acquisition/Rewet unter Druck:

- Acquisition Time 1 =: 23 s
- Acquisition Time 2 =: 48 s
- Acquisition Time 3 =: 62 s
- Rewet 1 =: < 0,1 g
- Rewet 2 =: 0,3 g
- Rewet 3 =: 1,5 g

### Beispiel 2:

In einem durch geschäumtes Kunststoffmaterial gut isolierten Polyethylengefäß mit einem Fassungsvermögen von 10 1 werden 3400 g entsalztes Wasser und 1350 g Acrylsäure vorgelegt. Nun erfolgt Zugabe von 11,5 g Allylmethacrylat als Copolymerisationsvernetzer und 270 g einer alkalischen Natriumaluminat-Lösung als Reaktivvernetzer, die rechnerisch 18,7 Gew.-% Al₂O₃ und 20 Gew.-% Na₂O enthält. Bei einer Temperatur von 4 °C werden die Initiatoren, bestehend aus 2,2 g 2,2'-Azobisamidino-propandihydrochlorid, gelöst in 20 g entsalztes Wasser, 4 g Kaliumperoxodisulfat, gelöst in 150 g entsalztes Wasser sowie 0,4 g Ascorbinsäure, gelöst in 20 g entsalztes Wasser, nacheinander zugegeben und verrührt. Die Reaktionslösung wird daraufhin ohne Rühren stehengelassen. Die Polymerisation verläuft adiabatisch, wobei die Temperatur bis auf ca. 90 °C ansteigt und ein festes Gel entsteht. Dieses wird anschließend mechanisch zerkleinert und durch Zugabe von 50 Gew.-%iger Natronlauge auf einen pH-Wert von 6,0 eingestellt. Das Gel wird dann getrocknet, gemahlen und auf eine Korngrößenverteilung von 100 - 850 µm abgesiebt. 1 kg dieses getrockneten Hydrogels wird in einem Pflugscharmischer mit einer Lösung bestehend aus 40 g entsalztes Wasser, 60 g i-Propanol und 30 g einer 15 Gew.-%igen wäßrigen Lösung eines Polyamidoamin-Epichlorhydrin-Adduktes (RETEN 204 LS der Fa. Hercules) besprüht und anschließend für 45 Minuten bei 160 °C getempert. Das vorliegend beschriebene Produkt zeigt folgende Eigenschaften:
- Pressure Absorbency Index =: 93,1
- Vertikal-Absorption unter Druck =: 14,0 g/g
- PUP 0.7 psi (4826,5 Pa) =: 21,7 g/g
- AUL 21000 dyn/cm² (2100 Pa) =: 25,4 g/g

### Acquisition/Rewet unter Druck:

- Acquisition Time 1 =: 24 s
- Acquisition Time 2 =: 51 s
- Acquisition Time 3 =: 70 s
- Rewet 1 =: < 0,1 g
- Rewet 2 =: 0,4 g
- Rewet 3 =: 1 , 8 g

### Beispiel 3:

In einem durch geschäumtes Kunststoffmaterial gut isolierten Polyethylengefäß mit einem Fassungsvermögen von 10 1 werden 3500 g entsalztes Wasser und 1500 g Acrylsäure vorgelegt. Nun erfolgt Zugabe von 7,5 g Methylenbisacrylamid als Copolymerisationsvernetzer und von 7 g 2-Hydroxyethylmethacrylat als gemischter Copolymerisations-/Reaktivvernetzer. Bei einer Temperatur von 2 °C werden die Initiatoren, bestehend aus 2,0 g 2,2'-Azobisamidinopropandihydrochlorid, gelöst in 20 g entsalztes Wasser, 4,4 g Kaliumperoxodisulfat, gelöst in 150 g entsalztes Wasser sowie 0,8 g Ascorbinsäure, gelöst in 20 g entsalztes Wasser, nacheinander zugegeben und verrührt. Die Reaktionslösung wird daraufhin ohne Rühren stehengelassen. Die Polymerisation verläuft adiabatisch, wobei die Temperatur bis auf ca. 98 °C ansteigt und ein festes Gel entsteht. Dieses wird anschließend mechanisch zerkleinert und durch Zugabe von 50 Gew.-%iger Natronlauge auf einen pH-Wert von 6,0 eingestellt. Das Gel wird dann getrocknet, gemahlen und auf eine Korngrößenverteilung von 100 - 850 µm abgesiebt. 600 g dieses getrockneten Hydrogels werden in einem Petterson & Kelly-Mischer mit einer Lösung bestehend aus 1 g Bisoxazolin, 1,2 g Aluminiumsulfat, 22,5 g i-Propanol und 22,5 g entsalztes Wasser besprüht und anschließend für 45 Minuten bei 185 °C getempert. Das vorliegend beschriebene Produkt zeigt folgende Eigenschaften:
- Pressure Absorbency Index =: 85,8
- Vertikal-Absorption unter Druck =: 17,2 g/g
- PUP 0.7 psi (4826,5 Pa) =: 21,3 g/g
- AUL 2100 dyn/cm² (2100 Pa) =: 23,6 g/g

### Acquisition/Rewet unter Druck:

- Acquisition Time 1 =: 22 s
- Acquisition Time 2 =: 45 s
- Acquisition Time 3 =: 58 s
- Rewet 1 =: < 0,1 g
- Rewet 2 =: 0,4 g
- Rewet 3 =: 1,6 g

### Beispiel 4:

In einem durch geschäumtes Kunststoffmaterial gut isolierten Polyethylengefäß mit einem Fassungsvermögen von 30 1 werden 14340 g entsalztes Wasser und 72 g Pentaerythritoltriallylether als Copolymerisationsvernetzer vorgelegt, 5172 g Natriumbicarbonat darin suspendiert und langsam 5990 g Acrylsäure so zudosiert, daß ein Überschäumen der Reaktionslösung vermieden wird, wobei sich diese auf eine Temperatur von ca. 3 - 5 °C abkühlt. Bei einer Temperatur von 4 °C werden die Initiatoren, 6,0 g 2,2'-Azobisamidinopropandihydrochlorid, gelöst in 60 g entsalztes Wasser, 12 g Kaliumperoxodisulfat, gelöst in 450 g entsalztes Wasser, sowie 1,2 g Ascorbinsäure, gelöst in 50 g entsalztes Wasser, nacheinander zugegeben und gut verrührt. Die Reaktionslösung wird daraufhin ohne Rühren stehen gelassen. Die Polymerisation erfolgt adiabatisch, wobei die Temperatur bis auf ca. 85 °C ansteigt und ein Gel entsteht. Dieses wird anschließend in einen Kneter überführt, mit 60 g Ethylenglykoldiglycidylether als Reaktivvernetzer, gelöst in 500 g entsalztes Wasser, versetzt, homogen verknetet, zerkleinert, im Luftstrom bei 170 °C getrocknet, gemahlen und gesiebt. 1 kg dieses Produktes wurde in einem Pflugscharmischer mit einer Lösung von 2 g Polyglycerinpolyglycidylether (Denacol EX-512 von Nagase Chemicals Ltd.), 0,3 g Zitronensäure, 60 g entsalztes Wasser und 40 g 1,2-Propandiol besprüht und anschließend 40 Minuten lang bei 150 °C getempert. Anschließend wurde das Produkt mit 0,1 Gew.-% hydrophilem Silica (Aerosil 200) abgemischt und die Kornfraktion 120 - 850 µm abgesiebt. Man erhielt ein Produkt, das gekennzeichnet ist durch folgende physikalischen Daten:
- Pressure Absorbency Index =: 96,2
- Vertikal-Absorption unter Druck =: 15,0 g/g
- PUP 0.7 psi (4826,5 Pa) =: 20,8 g/g
- AUL 2100 dyn/cm² (2100 Pa) =: 23,5 g/g

### Acquisition/Rewet unter Druck:

- Acquisition Time 1 =: 27 s
- Acquisition Time 2 =: 51 s
- Acquisition Time 3 =: 73 s
- Rewet 1 =: < 0,1 g
- Rewet 2 =: 0,5 g
- Rewet 3 =: 1,9 g

Die nach den Beispielen 1 bis 4 erhaltenen Hydrogele zeichnen sich durch hervorragendes Absorptionsvermögen bei gleichzeitig ausgezeichneter Flüssigkeitsweiterleitung aus und sind deshalb in hervorragender Weise als Absorptionsmittel für Wasser und wäßrige Flüssigkeiten, insbesondere von Körperflüssigkeiten, wie z. B. Urin oder Blut geeignet, beispielsweise in Hygieneartikeln wie z. B. Baby- und Erwachsenenwindeln, Damenbinden, Tampons und dergleichen.

### Vergleichsbeispiele:

### Vergleichsbeispiel 1:

Es wird wie in Beispiel 1 verfahren, jedoch wird dem Gel kein Polyamidoamin-Epichlorhydrin-Addukt als Reaktivvernetzer zugesetzt. Das vorliegend beschriebene Produkt zeigt folgende Eigenschaften:
- Pressure Absorbency Index =: 122,5
- Vertikal-Absorption unter Druck =: 9,0 g/g
- PUP 0.7 psi (4826,5 Pa) =: 30,5 g/g
- AUL 21000 dyn/cm² (2100 Pa) =: 32,5 g/g

### Acquisition/Rewet unter Druck:

- Acquisition Time 1 =: 29 s
- Acquisition Time 2 =: 88 s
- Acquisition Time 3 =: 142 s
- Rewet 1 =: < 0,1 g
- Rewet 2 =: 0,6 g
- Rewet 3 =: 2,9 g

### Vergleichsbeispiel 2:

Es wird wie in Beispiel 2 vorgegangen, jedoch wird der Monomerlösung kein Natriumaluminat als Reaktivvernetzer zugesetzt. Das vorliegend beschriebene Produkt zeigt folgende Eigenschaften:
- Pressure Absorbency Index =: 118,6
- Vertikal-Absorption unter Druck =: 8,0 g/g
- PUP 0,7 psi (4826,5 Pa) =: 31,8 g/g
- AUL 21000 dyn/cm² (2100 Pa) =: 33,7 g/g

### Acquisition/Rewet unter Druck:

- Acquisition Time 1 =: 32 s
- Acquisition Time 2 =: 95 s
- Acquisition Time 3 =: 166 s
- Rewet 1 =: < 0,1 g
- Rewet 2 =: 0,7 g
- Rewet 3 =: 3,3 g

### Vergleichsbeispiel 3:

Es wird wie in Beispiel 3 vorgegangen, jedoch erfolgt kein Zusatz von Methylenbisacrylamid als Copolymerisationvernetzer zur Monomerlösung. Das vorliegend beschriebene Produkt zeigt folgende Eigenschaften:
- Pressure Absorbency Index =: 82,5
- Vertikal-Absorption unter Druck =: 5,4 g/g
- PUP 0,7 psi (4826,5 Pa) =: 16,4 g/g
- AUL 2100 dyn/cm² (2100 Pa) =: 26,8 g/g

### Acquisition/Rewet unter Druck:

- Acquisition Time 1 =: 37 s
- Acquisition Time 2 =: 135 s
- Acquisition Time 3 =: 270 s
- Rewet 1 =: 0,2 g
- Rewet 2 =: 1,2 g
- Rewet 3 =: 5,4 g

### Vergleichsbeispiel 4:

Es wird wie in Beispiel 4 vorgegangen, jedoch dem Gel kein Ethylenglykoldiglycidylether als Reaktivvernetzer zugesetzt. Man erhielt ein Produkt, das gekennzeichnet ist durch folgende physikalischen Daten:
- Pressure Absorbency Index =: 127,4
- Vertikal-Absorption unter Druck =: 10,5 g/g
- PUP 0,7 psi (4826,5 Pa) =: 35,8 g/g
- AUL 2100 dyn/cm² (2100 Pa) =: 34,2 g/g

### Acquisition/Rewet unter Druck:

- Acquisition Time 1 =: 24 s
- Acquisition Time 2 =: 87 s
- Acquisition Time 3 =: 115 s
- Rewet 1 =: < 0,1 g
- Rewet 2 =: 0,5 g
- Rewet 3 =: 2,8 g

Die nach den Vergleichsbeispielen 1 bis 4 erhaltenen Hydrogele zeigen gegenüber den nach Beispielen 1 bis 4 erhaltenen Hydrogele betreffend Flüssigkeitstransport- und -weiterleitung deutliche Nachteile, ersichtlich in höheren Werten für Acquisition Time 3 und Rewet 3 im Acquisition/Rewet-Test unter Druck.

## Patentansprüche

1. Vernetztes, hydrophiles, hochquellfähiges Hydrogel auf Basis polymerisierter Monomerer oder auf Basis von Pfropfpolymeren, **dadurch gekennzeichnet, daß** es einen Pressure Absorbency Index < 100 und eine Vertikalabsorption unter Druck von 1922,8 Pa von mindestens 12 g/g besitzt.

2. Vernetztes, hydrophiles, hochquellfähiges Hydrogel gemäß Anspruch 1, **dadurch gekennzeichnet, daß** es eine Performance Under Pressure unter einer Druckbelastung von 4826,5 Pa von < 23 g/g aufweist.

3. Vernetztes, hydrophiles, hochquellfähiges Hydrogel gemäß Anspruche 1 oder 2, **dadurch gekennzeichnet, daß** es eine Absorbency Under Load unter einer Druckbelastung von 2100 Pa von < 27 g/g aufweist.

4. Hochvernetztes, hydrophiles, hochquellfähiges Hydrogel gemäß einem der Ansprüche 1 bis 3, **dadurch gekennzeichnet, daß** das hydrophile Monomere Verbindungen der allgemeinen Formel 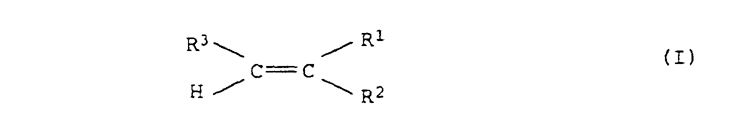 bedeutet, worin
R¹ Wasserstoff, Methyl oder Ethyl,
R² die Gruppe -COOR⁴, die Sulfonylgruppe, die Phosphonylgruppe, die mit (C₁-C₄)-Alkanol veresterte Phosphonylgruppe oder eine Gruppe der Formel 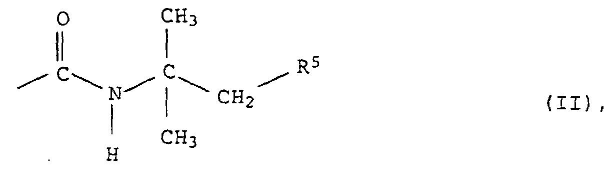 in der
R³ Wasserstoff, Methyl, Ethyl oder die Carboxylgruppe,
R⁴ Wasserstoff, Alkalimetall- oder Ammoniumion und
R⁵ Sulfonyl-, Phosphonyl- oder Carboxylgruppen sind.

5. Vernetztes, hydrophiles, hochquellfähiges Hydrogel gemäß einem oder mehreren der Ansprüche 1 bis 4, **dadurch gekennzeichnet, daß** die Pfropfgrundlage mindestens eine Verbindung aus der Gruppe Stärke, Stärkederivate, Cellulose, Cellulosederivate, Polyvinylalkohol, Polyalkylenoxid, Polyethylenoxid, Polypropylenoxid und hydrophile Polyester sind.

6. Verfahren zur Herstellung der vernetzten, hydrophilen, hochquellfähigen Hydrogele nach einem der Ansprüche 1 bis 5, **dadurch gekennzeichnet, daß** man Säuregruppen enthaltende hydrophile Monomere, deren Alkalimetall- oder Ammoniumsalze mit
(a) einem Copolymerisationsvernetzer, der mindestens zwei ethylenisch ungesättigte Doppelbindungen im Molekül enthält, und
(b) einem Reaktivvernetzer A, der eine ethylenisch ungesättigte Doppelbindung und mindestens eine funktionelle Gruppe aufweist, die mit den Säuregruppen der hydrophilen Polymerisate kovalente Bindungen bildet, einem Reaktivvernetzer B, der mindestens zwei funktionelle Gruppen aufweist, die mit den Säuregruppen der hydrophilen Polymerisate kovalente Bindungen bilden, und/oder mit Ionen mehrwertiger Metalle
gegebenenfalls in Gegenwart mindestens einer Pfropfgrundlage, radikalisch zu einem vernetzten, hydrophilen Grundpolymer polymerisiert, das Grundpolymer zerkleinert und die Oberfläche der Partikeln des Grundpolymers nachvernetzt.

7. Verfahren zur Herstellung der vernetzten, hydrophilen, hochquellfähigen Hydrogele nach einem der Ansprüche 1 bis 5, **dadurch gekennzeichnet, daß** man Säuregruppen enthaltende hydrophile Monomere, deren Alkalimetall- oder Ammoniumsalze mit
(a) einem Copolymerisationsvernetzer, der mindestens zwei ethylenisch ungesättigte Doppelbindungen im Molekül enthält, und gegebenenfalls
(b) einem Reaktivvernetzer A, der eine ethylenisch ungesättigte Doppelbindung und mindestens eine funktionelle Gruppe aufweist, die mit den Säuregruppen der hydrophilen Polymerisate kovalente Bindungen bildet,
gegebenenfalls in Gegenwart einer Pfropfgrundlage, radikalisch zu einem vernetzten, hydrophilen Grundpolymer polymerisiert, das Grundpolymer zerkleinert, es während des Zerkleinerns oder danach mit einem Reaktivvernetzer B und/oder mit Ionen mehrwertiger Metalle mischt und die Oberfläche der Partikeln des Grundpolymers nachvernetzt.

8. Verfahren nach Anspruch 6 oder 7, **dadurch gekennzeichnet, daß** man als Copolymerisationsvernetzer Verbindungen mit mindestens zwei Allyl-, Methacrylat- und/oder Acrylatgruppen einsetzt.

9. Verfahren nach Anspruch 6 oder 7, **dadurch gekennzeichnet, daß** man als Reaktivvernetzer B Polyamidoamine, deren Umsetzungsprodukte mit Epichlorhydrin oder Bischlorhydrinethern von Alkylenglykolen oder Polyalkylenglykolen einsetzt.

10. Verfahren nach Anspruch 6 oder 7, **dadurch gekennzeichnet, daß** man daß man als Vernetzer (b) Natriumaluminat einsetzt.

11. Verfahren nach Anspruch 6 oder 7, **dadurch gekennzeichnet, daß** man die Copolymerisation der Säuregruppen enthaltenden hydrophilen Monomeren in Gegenwart von
(a) Allylmethacrylat, Tetraallyloxethan, Methylenbisacrylamid, Petaerythrittriallylether oder deren Mischungen und
(b) Hydroxyethylacrylat, Hydroxyethylmethacrylat, Hydroxypropylacrylat, Hydroxypropylmethacrylat, Hydroxybutylacrylaten oder Mischungen der genannten Verbindungen
als Vernetzer durchführt.

12. Verfahren nach Anspruch 7, **dadurch gekennzeichnet, daß** man zur Nachvernetzung der Oberfläche der Partikeln des Grundpolymers Natriumaluminat einsetzt.

13. Verfahren nach Anspruch 6 oder 7, **dadurch gekennzeichnet, daß** man die Nachvernetzung der Oberfläche der teilchenförmigen, hydrophilen Grundpolymere mit Diglycidylethern, Umsetzungsprodukten von Polyamidoaminen mit Epichlorhydrin, Bischlorhyrinethern von Alkylenglykolen oder Polyalkylenglykolen, Polyethyleniminen, Vinylamineinheiten enthaltenden Polymeren oder deren Mischungen durchführt.

14. Verfahren nach Anspruch 6 oder 7, **dadurch gekennzeichnet, daß** man das vernetzte, hydrophile Grundpolymere in Gegenwart von
(a) mindestens einem Copolymerisationsvernetzer und
(b) mindestens einem Reaktivvernetzer B herstellt.

15. Verwendung der vernetzten, hydrophilen, hochquellfähigen Hydrogele nach den Ansprüchen 1 bis 5 als Absorptionsmittel für Wasser und wäßrige Flüssigkeiten.

16. Verwendung nach Anspruch 15, **dadurch gekennzeichnet, daß** man die vernetzten, hydrophilen, hochquellfähigen Hydrogele zur Absorption von Körperflüssigkeiten in Hygieneartikeln einsetzt.

## Claims

1. A crosslinked, hydrophilic, highly swellable hydrogel based on polymerized monomers or based on graft polymers, **characterized by** a Pressure Absorbency Index < 100 and a Vertical Absorption of not less than 12 g/g under a pressure of 1922.8 Pa.

2. A crosslinked, hydrophilic, highly swellable hydrogel as claimed in claim 1, **characterized by** a Performance Under Pressure value of < 23 g/g under a confining pressure of 4826.5 Pa.

3. A crosslinked, hydrophilic, highly swellable hydrogel as claimed in claim 1 or 2, **characterized by** an Absorbency Under Load value of < 27 g/g under a confining pressure of 2100 Pa.

4. A highly crosslinked, hydrophilic, highly swellable hydrogel as claimed in any of claims 1 to 3, wherein the hydrophilic monomer comprises compounds of the general formula 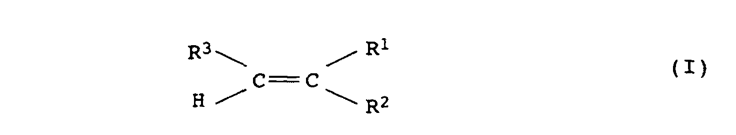 where
R¹ is hydrogen, methyl or ethyl,
R² is a -COOR⁴ group, a sulfonyl group, a phosphonyl group, a phosphonyl group esterified with (C₁-C₄) alkanol or a group of the formula 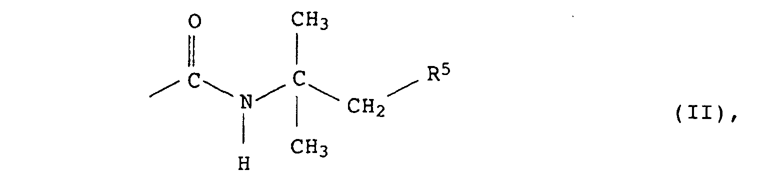 where
R³ is hydrogen, methyl, ethyl or carboxyl,
R⁴ is hydrogen, an alkali metal ion or an ammonium ion, and
R⁵ is sulfonyl, phosphonyl or carboxyl.

5. A crosslinked, hydrophilic, highly swellable hydrogel as claimed in one or more of claims 1 to 4, wherein the grafting base is at least one compound selected from the group consisting of starch, starch derivatives, cellulose, cellulose derivatives, polyvinyl alcohol, polyalkylene oxide, polyethylene oxide, polypropylene oxide and hydrophilic polyesters.

6. A process for producing the crosslinked, hydrophilic, highly swellable hydrogels of any of claims 1 to 5, which comprises free-radically polymerizing hydrophilic monomers which contain acid groups, or their alkali metal or ammonium salts, with
(a) a copolymerization crosslinker which contains at least two ethylenically unsaturated double bonds in the molecule, and
(b) a reactive crosslinker A which contains an ethylenically unsaturated double bond and at least one functional group capable of forming covalent bonds with the acid groups of the hydrophilic polymers, a reactive crosslinker B which contains at least two functional groups capable of forming covalent bonds with the acid groups of the hydrophilic polymers, and/or with ions of polyvalent metals
optionally in the presence of at least one grafting base to form a crosslinked, hydrophilic base polymer, comminuting said base polymer and postcrosslinking the surface of the particles of said base polymer.

7. A process for producing the crosslinked, hydrophilic, highly swellable hydrogels of any of claims 1 to 5, which comprises free-radically polymerizing hydrophilic monomers which contain acid groups, or their alkali metal or ammonium salts, with
(a) a copolymerization crosslinker which contains at least two ethylenically unsaturated double bonds in the molecule, and optionally
(b) a reactive crosslinker A which contains an ethylenically unsaturated double bond and at least one functional group capable of forming covalent bonds with the acid groups of the hydrophilic polymers,
optionally in the presence of a grafting base to form a crosslinked, hydrophilic base polymer, comminuting said base polymer, during said comminuting or thereafter mixing it with a reactive crosslinker B and/or with ions of polyvalent metals and postcrosslinking the surface of the particles of said base polymer.

8. A process as claimed in claim 6 or 7, wherein the copolymerization crosslinker used is a compound having at least two allyl, methacrylate and/or acrylate groups.

9. A process as claimed in claim 6 or 7, wherein said reactive crosslinker B is a polyamidoamine or its reaction product with epichlorohydrin or bischlorohydrin ethers of alkylene glycols or polyalkylene glycols.

10. A process as claimed in claim 6 or 7, wherein crosslinker (b) is sodium aluminate.

11. A process as claimed in claim 6 or 7, wherein the copolymerization of the hydrophilic monomers which contain acid groups is carried out in the presence of
(a) allyl methacrylate, tetraallyloxyethane, methylenebisacrylamide, pentaerythritol triallyl ether or mixtures thereof, and
(b) hydroxyethyl acrylate, hydroxyethyl methacrylate, hydroxypropyl acrylate, hydroxypropyl methacrylate, hydroxybutyl acrylates or mixtures thereof
as crosslinker.

12. A process as claimed in claim 7, wherein said postcrosslinking of said surface of said particles of said base polymer is effected using sodium aluminate.

13. A process as claimed in claim 6 or 7, wherein said postcrosslinking of said surface of the particulate hydrophilic base polymers is effected using diglycidyl ethers, reaction products of polyamidoamines with epichlorohydrin, bischlorohydrin ethers of alkylene glycols or polyalkylene glycols, polyethyleneimines, polymers containing vinylamine units or mixtures thereof.

14. A process as claimed in claim 6 or 7, wherein said crosslinked, hydrophilic base polymer is produced in the presence of
(a) at least one copolymerization crosslinker and
(b) at least one reactive crosslinker B.

15. The use of the crosslinked, hydrophilic, highly swellable hydrogels of any of claims 1 to 5 as absorbents for water and aqueous fluids.

16. The use of claim 15, whereby the crosslinked, hydrophilic, highly swellable hydrogels are used for absorbing body fluids in hygiene articles.

## Revendications

1. Hydrogel réticulé, hydrophile, à forte capacité de gonflement à base de monomères polymérisés ou à base de polymères de greffage, **caractérisé en ce qu'**il possède un Pressure Absorbency Index < 100 et une absorption verticale, sous pression de 1922,8 Pa, d'au moins 12 g/g.

2. Hydrogel réticulé, hydrophile, à forte capacité de gonflement suivant la revendication 1, **caractérisé en ce qu'**il présente une Performance Under Pressure, sous une charge de pression de 4826,5 Pa, de < 23 g/g.

3. Hydrogel réticulé, hydrophile, à forte capacité de gonflement suivant l'une des revendications 1 et 2, **caractérisé en ce qu'**il présente une Absorbency Under Load, sous une charge de pression de 2100 Pa, de < 27 g/g.

4. Hydrogel fortement réticulé, hydrophile, à forte capacité de gonflement, suivant l'une des revendications 1 à 3, **caractérisé en ce que** le monomère hydrophile représente des composés de la formule générale : 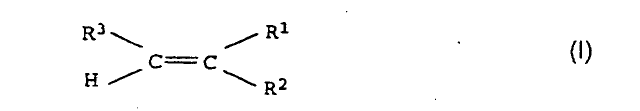 dans laquelle
R¹ représente de l'hydrogène, du méthyle ou de l'éthyle,
R² représente le groupe -COOR⁴, le groupe sulfonyle, le groupe phosphonyle, le groupe phosphonyle estérifié par un alcanol en C₁-C₄ ou un groupe de la formule : 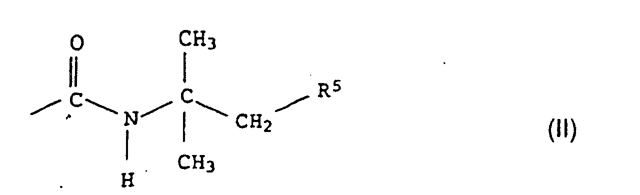 dans laquelle
R³ représente de l'hydrogène, du méthyle, de l'éthyle ou le groupe carboxyle,
R⁴ représente de l'hydrogène, ou un ion de métal alcalin ou d'ammonium, et
R⁵ représente des groupes sulfonyle, phosphonyle ou carboxyle.

5. Hydrogel réticulé, hydrophile, à forte capacité de gonflement suivant une ou plusieurs des revendications 1 à 4, **caractérisé en ce que** la base de greffage est au moins un composé du groupe comprenant de l'amidon, des dérivés d'amidon, de la cellulose, des dérivés de cellulose, de l'alcool polyvinylique, de l'oxyde de polyalkylène, de l'oxyde de polyéthylène, de l'oxyde de polypropylène et des polyesters hydrophiles.

6. Procédé de préparation des hydrogels, réticulés, hydrophiles, à forte capacité de gonflement suivant l'une des revendications 1 à 5, **caractérisé en ce qu'**on polymérise des monomères hydrophiles contenant des groupes acides, leurs sels de métal alcalin ou d'ammonium, de manière radicalaire avec
(a) un agent réticulant de copolymérisation, qui contient au moins deux doubles liaisons éthyléniquement insaturées dans la molécule, et
(b) un agent réticulant réactif A, qui présente une double liaison éthyléniquement insaturée et au moins un groupe fonctionnel, qui forme, avec les groupes acides des polymères hydrophiles, des liaisons covalentes, un agent réticulant réactif B, qui présente au moins deux groupes fonctionnels qui forment, avec les groupes acides des polymères hydrophiles, des liaisons covalentes, et/ou des ions de métaux plurivalents,
éventuellement en présence d'au moins une base de greffage, ce qui donne un polymère de base réticulé, hydrophile, **en ce qu'**on fragmente le polymère de base et **en ce qu'**on poursuit la réticulation de la surface des particules des polymères de base.

7. Procédé de préparation des hydrogels réticulés, hydrophiles, à forte capacité de gonflement suivant l'une des revendications 1 à 5, **caractérisé** en qu'on polymérise des monomères hydrophiles contenant des groupes acides, leurs sels de métal alcalin ou d'ammonium, de manière radicalaire avec
(a) un agent réticulant de copolymérisation, qui contient au moins deux doubles liaisons éthyléniquement insaturées dans la molécule, et éventuellement
(b) un agent réticulant réactif A, qui présente une double liaison éthyléniquement insaturée et au moins un groupe fonctionnel, qui forme, avec les groupes acides des polymères hydrophiles, des liaisons covalentes,
éventuellement en présence d'une base de greffage, ce qui donne un polymère de base réticulé, hydrophile, en ce qu'on fragmente le polymère de base, en ce qu'on le mélange pendant la fragmentation ou après elle avec un agent réticulant réactif B et/ou avec des ions de métaux plurivalents et en ce qu'on poursuit la réticulation de la surface des particules du polymère de base.

8. Procédé suivant l'une des revendications 6 et 7, **caractérisé en ce que**, comme agent réticulant de polymérisation, on met en oeuvre des composés ayant au moins deux groupes allyle, méthacrylate et/ou acrylate.

9. Procédé suivant l'une des revendications 6 et 7, **caractérisé en ce que**, comme agent réticulant réactif B, on met en oeuvre des polyamidoamines, leurs produits de réaction avec de l'épichlorhydrine ou des éthers de bischlorhydrine d'alkylèneglycols ou de polyalkylèneglycols.

10. Procédé suivant l'une des revendications 6 et 7, **caractérisé en ce qu'**on met en oeuvre, comme agent réticulant (b), de l'aluminate de sodium.

11. Procédé suivant l'une des revendications 6 et 7, **caractérisé en ce qu'**on effectue la copolymérisation des monomères hydrophiles contenant des groupes acides en présence
(a) de méthacrylate d'allyle, de tétraallyloxéthane, de méthylènebisacrylamide, d'éther triallylylique de pentaérythrite ou de leurs mélanges, et
(b) d'acrylate d'hydroxyéthyle, de méthacrylate d'hydroxyéthyle, d'acrylate d'hydroxypropyle, de méthacrylate d'hydroxypropyle, d'acrylate d'hydroxybutyle ou de mélanges des composés cités,
à titre d'agents réticulants.

12. Procédé suivant la revendication 7, **caractérisé en ce que**, pour la poursuite de la réticulation des surfaces des particules du polymère de base, on met en oeuvre de l'aluminate de sodium.

13. Procédé suivant l'une des revendications 6 et 7, **caractérisé en ce qu'**on effectue la poursuite de réticulation des surfaces des polymères de base hydrophiles en forme de particules avec des éthers diglycidyliques, des produits de réaction de polyamidoamines avec de l'épichlorhydrine, des éthers de bischlorhydrine d'alkylèneglycols ou de polyalkylèneglycols, des polyéthylèneimines, de polymères contenant des unités vinylamine ou leurs mélanges.

14. Procédé suivant l'une des revendications 6 et 7, **caractérisé en ce qu'**on prépare le polymère de base réticulé, hydrophile, en présence
(a) d'au moins un agent réticulant de copolymérisation, et
(b) d'au moins un agent réticulant réactif B.

15. Utilisation des hydrogels réticulés, hydrophiles, à forte capacité de gonflement, suivant l'une des revendications 1 à 5, comme agent d'absorption pour de l'eau et des liquides aqueux.

16. Utilisation suivant la revendication 5, **caractérisée en ce qu'**on met en oeuvre les hydrogels réticulés, hydrophiles, à forte capacité de gonflement pour l'absorption de liquides corporels dans des articles d'hygiène.
